# EUROPEAN PATENT SPECIFICATION

(11) **EP 4 436 644 B1**
(45) Date of publication and mention of the grant of the patent: **17.09.2025**
(21) Application number: 22822281.6
(22) Date of filing: 10.11.2022
(51) Int. Cl.: A61M 16/00, A61M 16/20

(54) **SYSTEM FOR VENTILATING A PATIENT**
SYSTEM ZUR BEATMUNG EINES PATIENTEN
SYSTÈME DE VENTILATION D'UN PATIENT

(30) Priority: 10.11.2021 BE 202105875
(43) Date of publication of application: 02.10.2024
(73) Proprietor: Medec International BV, 9300 Aalst (BE)
(72) Inventor: BRAEM, Kristof, 9320 Nieuwerkerken-Aalst (BE); MULIER, Jan P., 3018 Wijgmaal (BE)
(74) Representative: Arnold & Siedsma
(86) International application number: PCT/EP2022/081520
(87) International publication number: WO 2023/083981

(56) References cited:
- WO-A1-2010/108552
- US-A- 5 797 393
- US-A1- 2003 168 066
- US-A1- 2010 252 046
- US-B2- 9 492 629

## Description

### Technical field

The invention relates to mechanical ventilation of patients. In particular, the invention relates to an improved respiratory actuation of a patient, being connected to a mechanical ventilator or breathing apparatus, based on controlling the expiration or expiratory flow during the expiratory phase. The improved respiratory actuation being preferably balanced by means of inducing a constant expiratory flow being equal to a constant inspiratory flow.

### Background of the invention

During anaesthesia or when patients cannot breathe on their own due to a critical illness, they are mechanically ventilated. Many different ventilation modes exist, controlling different ventilatory parameters such as for example tidal volume, i.e. the amount of air that moves in or out of the lungs with each respiratory cycle, and patient pressure when using common positive-pressure ventilators. Conventional modes such as Volume Controlled Ventilation (VCV) or Pressure Controlled Ventilation (PCV) are widely used and well-understood, but often they fail to match specific patient based requirements. For instance, failure may occur in case of mechanical ventilation of patients who suffer from obesity. Due to their large abdominal fat mass, the transition from inspiratory phase to expiratory phase can suddenly cause a significant change in respiratory flow and may affect the pulmonary compliance. As a consequence, this can create ventilatory complications such as atelectasis.

The significant change in respiratory flow is caused by the working principle of mechanical ventilation when using positive pressure ventilators, wherein a positive pressure is applied during the inspiratory phase to the patient. When the set volume or target pressure is reached, the artificial breath will be terminated. Subsequently, the airway pressure drops to zero (or the set PEEP (Positive End Expiratory Pressure) level if applicable) and the elastic recoil of the chest wall and lungs will push the tidal volume out in a passive way.

A system for providing a balanced respiratory actuation of a patient is disclosed in US Patent application publication 2003/168066.

### Aim of the invention

The aim of the invention is to mitigate the sudden and significant change in respiratory flow during transition from inspiration to expiration, by providing a method to control the respiratory system, in particular, controlling expiration during the expiratory phase thereof.

The aim of the invention is also to avoid sudden changes in the patient pressure, and by extension in the airway pressure during the expiratory phase, and hence instead letting the pressure decrease in a balanced way during this phase.

### Summary of the technology

The invention provides a system for providing a balanced respiratory actuation of a patient, being connected to a respiratory apparatus for breathing, the respiratory actuation comprising an inspiratory and an expiratory phase during which respectively inspiration and expiration of the patient takes place, wherein the system comprises a controller for executing a control algorithm (i) monitoring the inspiratory airway pressure and herewith defining a trajectory for the inspiratory phase, and (ii) based thereon, dynamically adjusting the flow (towards a constant level) of pressurized air in the expiratory line, herewith controlling the lung or airway pressure towards a predetermined trajectory, wherein said predetermined trajectory being based on the monitored inspiratory airway pressure and resembling in shape the trajectory of the inspiratory phase. The term balanced herewith refers to the fact that the curves, trajectory or profile (e.g. representing flow or pressure) of inspiratory and expiratory phase respectively, are evolving for example according to the same linearity or linear relation, i.e. they are appearing in relation to each other, in particular they are resembling or being comparable up to similar or equal in shape, although this does not necessarily imply that the curves, trajectory or profile are having the same slope. A distinction can be made amongst less and more perfect balance in approaching less or more a similar slope, linearity or e.g. curve angle. By means of example, whenever the (pressure) curve is nearly an isosceles triangle and/or the (flow) curve is representing a block wave, we could say that a rather perfect balance is acquired.

Dynamically adjusting the flow of pressurized air in the expiratory line may be performed by pneumatic means and/or electronic means.

Dynamically adjusting the flow of pressurized air in the expiratory line may be performed by means of controlling the position of a (pneumatically controlled) membrane provided therein (i.e. in the expiratory line), or based on the actuation of a proportional valve.

In an embodiment, the controller for executing a control algorithm is also taking measurements during the expiration phase, and said dynamically adjusting the flow of pressurized air in the expiratory line is based on said expiration measurements.

In an embodiment, the controller for executing a control algorithm starts after a set and/or computed time period and/or a certain condition is satisfied after the inspiration ends.

In an embodiment, the controller for executing a control algorithm stops after a set and/or computed time period and/or a certain condition is satisfied after the inspiration ends.

In an embodiment, the lung or airway pressure being controlled towards a predetermined trajectory, is characterized by a linear decrease.

The technology provides a method (not being part of the claimed invention) for providing a balanced respiratory actuation of a patient, being connected (or connectable) to a respiratory apparatus for breathing, the respiratory actuation comprising an inspiratory and an expiratory phase during which respectively inspiration and expiration of the patient takes place. The method comprises the step of, during the expiratory phase, controlling the expiration. Further, the method comprises the step of, during the inspiratory phase, monitoring the inspiratory behaviour, e.g. monitoring the inspiratory air (or airway) pressure. Herewith, controlling the expiration during the expiratory phase is based on the monitored inspiratory behaviour. In addition, controlling the expiration during the expiratory phase is performed by means of limiting or restricting the expiratory flow in order to (and hence going along with) controlling the (lung or airway) pressure towards a predetermined trajectory, which is based on the monitored inspiratory behaviour, e.g. on the monitored inspiratory air (or airway) pressure, and moreover, this predetermined trajectory is resembling in shape to the trajectory of the monitored inspiratory behaviour, e.g. of the monitored inspiratory air (or airway) pressure.

The respiratory behaviour may be represented either by the airway flow or by the airway pressure.

The respiratory actuation may mitigate the sudden change in airway flow (which otherwise typically occurs as known from the art) during transition from inspiration to expiration.

The airway pressure during the expiratory phase may be linearly decreased, possibly in a perfectly balanced way - herewith for example the obtained pressure curve per breath is nearly an isosceles triangle and/or the to be obtained flow curve is representing a block wave - for providing balanced respiratory actuation.

The maximum expiratory flow may be approximately the same as the maximum of the monitored inspiratory flow.

Controlling the expiration may be performed by pneumatic means and/or electronic means. Moreover, controlling the expiration may be executed by means of providing control signals to a pneumatically controlled membrane, being part of the expiratory line. Further, controlling the expiration may be based on a dynamically adjusted flow of pressurized air.

In an example, the step of controlling the expiration only starts after a predetermined amount of inspiration/expiration cycles.

In an example, during the expiration, measurements are taken, and controlling the expiration is based on such expiration measurements. In an example, prior to the use of such expiration measurements for controlling the expiration, one or more measurement manipulations such as filtering and/or computing of an average is performed.

In an example, controlling the expiration starts after a predetermined and/or computed time period and/or a certain condition is satisfied after the inspiration ends.

In an example, controlling the expiration stops after a predetermined and/or computed time period and/or a certain condition is satisfied after the inspiration ends.

In a further aspect, the technology provides a method (not being part of the claimed invention) for providing an improved, possibly balanced, respiratory actuation of a patient, possibly an unconscious patient undergoing anaesthesia, wherein the patient being connected to a respiratory apparatus for breathing for example during anaesthesia. The respiratory actuation comprises an inspiratory and an expiratory phase during which respectively inspiration and expiration of the patient takes place. The method comprises the step of (also) during (essentially the whole of) the expiratory phase, controlling the expiration (by limiting the expiratory flow). In general, the inspiratory phase is always controlled in some way, due to the active ventilation or supply of pressurized air to the patient. Moreover, the controlling of the expiration is possibly during essentially the whole of the expiratory phase, meaning for as good as whole but not necessarily during the entire expiratory phase. It may be for example that this controlling of the expiration is applied for a predetermined percentage of the expiratory phase instead. The respiratory behaviour, i.e. behaviour of the respiratory actuation, be it inspiratory or expiratory, may be represented either by the airway flow (e.g. in litres/minute) or by the airway pressure (e.g. in hPa, mbar, cm H2O).

The controlling of the expiration is for example done by limiting the expiratory flow. Usually, the expiratory flow is rather large as compared to the inspiratory flow. This means that, when considering the airway flow representation, a significant and sudden change in airway flow can be observed from inspiration to expiration. The improved respiratory actuation may mitigate such significant and sudden (default) change in airway flow during transition from inspiration to expiration. In case of patients suffering from obesities, the large expiratory flow can be very uncomfortable or even harming the patient during the respiratory actuation. In the aim of limiting the expiratory flow, and herewith approaching a flow which is comparable (in amount) with the inspiratory flow, the respiratory actuation is getting balanced, or in other words, a balanced flow is achieved. Hence, in an example, the airway pressure during the expiratory phase being decreased in a balanced way, for providing improved balanced respiratory actuation. Moreover, the to be obtained pressure curve per breath may be nearly an isosceles triangle.

According to an example, the method further comprises the step of during the inspiratory phase, monitoring the inspiratory behaviour. The controlling of the expiration during the expiratory phase can then be based on the monitored inspiratory behaviour. This kind of control can be seen as at least feedforward control. In an example, the maximum expiratory flow is approximately the same as the maximum of the monitored inspiratory flow.

The controlling of the expiration may be performed by pneumatic and/or electronic means. The controlling of the expiration is for example done by providing control signals to (dynamically adjust the position of) a pneumatically controlled membrane, being part of (at the end of) the expiratory line. The controlling the expiration may be based on a dynamically adjusted flow of pressurized air. The controlling the expiration may only start after a predetermined amount of inspiration/expiration cycles.

According to an example, during the (discrete) expiration, measurements (at discrete time instances) are taken. The controlling of the expiration can then be (further) based on the expiration measurements. This kind of control can be seen as additional feedback control. Prior to the use of the expiration measurements for controlling the expiration, one or more measurement manipulations such as (maximally) filtering and/or computing of a (moving) average is performed.

In an example, the controlling of the expiration may start after a predetermined and/or computed time period and/or a certain condition (determined from the measurements) is satisfied after the inspiration ends.

In an example, the controlling of the expiration may stop after a predetermined and/or computed time period and/or a certain condition (determined from the measurements) is satisfied after the inspiration ends.

In a further aspect of the technology, a method (not being part of the claimed invention) is provided for (automatically) activating any of the previous methods based on (discrete) measurements (at discrete time instances) taken during inspiration and/or expiration.

In a further aspect of the technology, a system is provided for providing an improved, possibly balanced, respiratory actuation of a patient, being connected to a respiratory apparatus for breathing. The respiratory actuation comprises an inspiratory and an expiratory phase during which respectively inspiration and expiration of the patient takes place. The system comprises a control algorithm (i) monitoring the inspiratory airway pressure, and (ii) based thereon, dynamically adjusting the flow of pressurized air in the expiratory line by (pneumatically and/or electronically) controlling the position of a membrane provided (at the end) therein.

In a further aspect, the technology also provides a method (not being part of the claimed invention) for calibrating any of the methods above, wherein the calibrating is determining any of the control parameters thereof.

### Brief description of the drawings

Figure 1 illustrates a graphical representation of the respiratory actuation of a patient as according to the art, wherein no active control is represented during the expiratory phase.
Figure 2 shows an embodiment of the improved respiratory actuation of a patient, illustrating active control during the expiratory phase, in accordance with the invention.
Figure 3 shows an embodiment of the improved respiratory actuation of a patient, taking into account delayed start of the active control, in accordance with the invention.
Figure 4 shows an embodiment of the improved respiratory actuation of a patient, taking into account delayed start of the active control and end pressure control, in accordance with the invention.
Figure 5 shows an embodiment of the improved (balanced) flow under active control, based on inspiratory phase monitoring, in accordance with the invention
Figure 6 shows an embodiment of the improved (balanced) flow under active control, illustrating the feedforward and the feedback controller combination, in accordance with the invention.
Figure 7 shows an embodiment of the improved respiratory actuation of a patient, illustrating a predetermined amount of inspiration/expiration cycles, in accordance with the invention
Figure 8 illustrates airway flow and corresponding pressure graph in case of volume controlled ventilation at constant flow as according to the art.
Figure 9 shows an embodiment of the balanced expiratory ventilation in accordance with the invention

### Detailed description of the invention

The invention relates to improved mechanical ventilation of patients, based on controlled and balanced expiratory ventilation. Herewith is meant that the expiration or expiratory flow during the expiratory phase being controlled, and this preferably by means of having the airway pressure decreased in a balanced way during this expiratory phase.

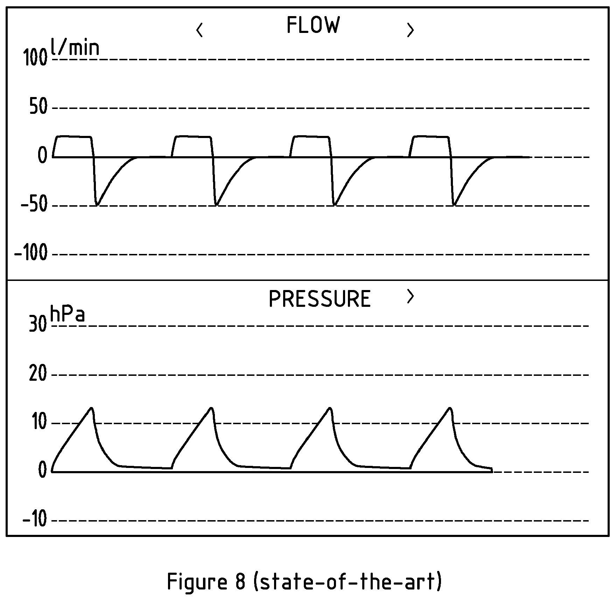

In accordance with the art, during volume controlled ventilation at constant flow, a constant flow can be calculated based on the set tidal volume and respiratory rate. As illustrated in Figure 8, during inspiration the flow remains constant on approximately 25 litres per minute and the airway pressure builds up linearly. When the set tidal volume is reached, the inspiration or inspiratory phase stops. At the start of expiration, a large expiratory flow is created resulting in a steep, exponential decrease of the pressure.

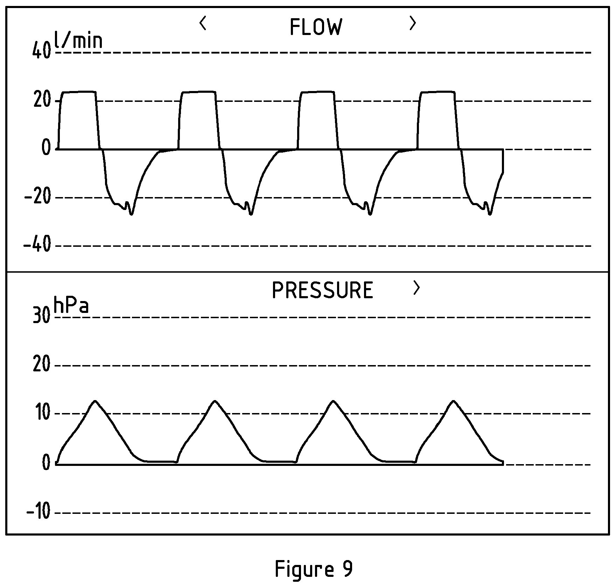

The goal of the controlled and balanced expiratory ventilation according to the invention, is to ensure a controlled expiratory phase in order to mitigate the sudden change in flow when changing from inspiration to expiration. On the other hand, the target may also be to let the pressure during expiration or the expiratory phase decrease in a balanced way. Figure 9 illustrates an embodiment showing the effect of applying this balanced and controlled outflow of air in the expiratory phase. It is clear that the expiratory flow is lowered to approximately the same as the inspiratory flow. The pressure curve per breath is nearly a isosceles triangle thanks to the controlled expiration. The pressure linearly increases for a certain amount at inspiratory phase, and then linearly decreases at expiratory phase for approximately the same amount and at the same pace, resulting in a balance as represented by the triangle. Thanks to this balanced expiratory ventilation, the lungs can return to their original state more gently and controlled. This may lead to a more calm breathing pattern of the patient and less ventilatory complications.

In order to achieve the controlled and balanced expiratory ventilation mode in accordance with an embodiment of the invention, the expiratory flow of air is controlled thanks to a pneumatically controlled membrane at the end of the expiratory line. The position of the membrane can be controlled based on a dynamically adjusted flow of pressurized air. The latter can be created thanks to a software algorithm checking at all stages the inspiratory airway pressure.

Throughout the description the definitions of curve, trajectory or profile (of the pressure and in particular of the flow) are used as alternatives.

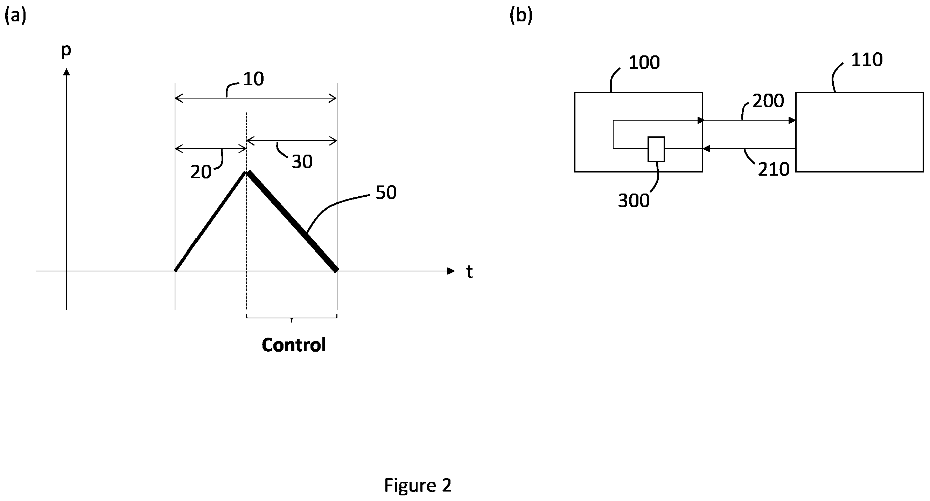

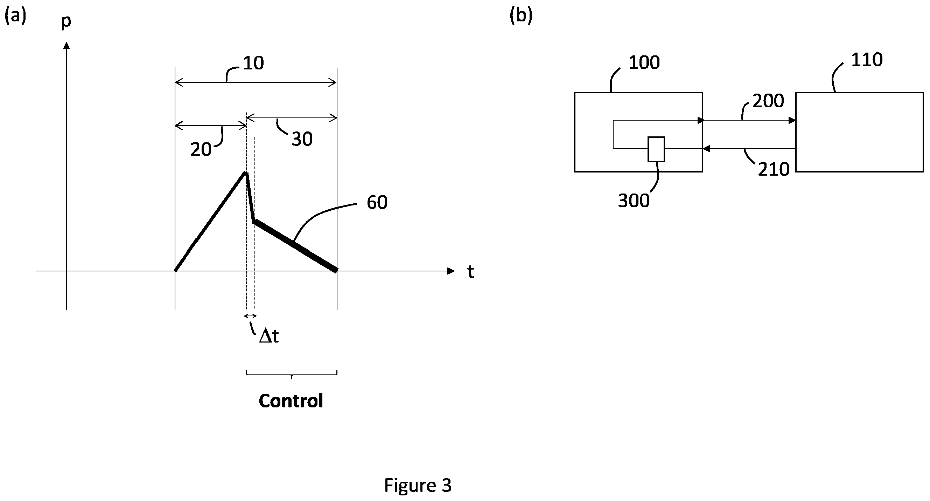

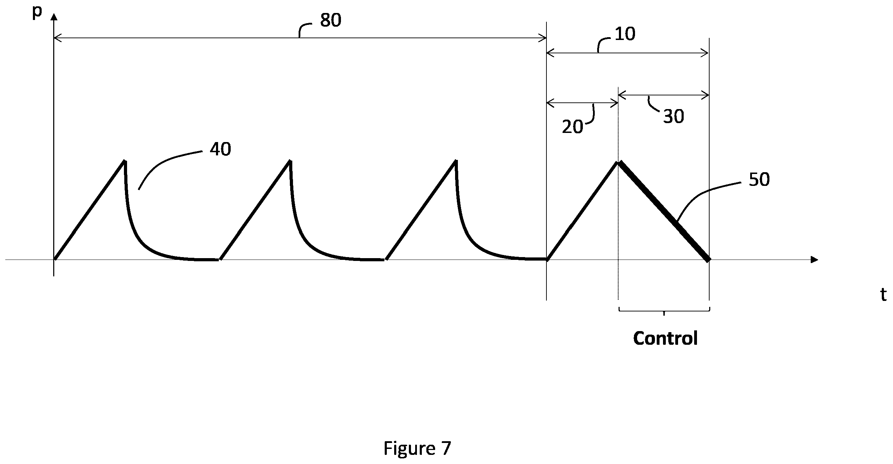

In the invention a so-called balanced implementation is chosen wherein the curve, trajectory or profile during expiration is based on or inspired by the curve, trajectory or profile of the inspiration, and being enforced by control actions. This at least in regime operation, for which can be referred to for example the embodiment of Figure 7, and/or subject to a time off-set, as for example depicted in Figure 3. In particular the shape of the two curves, i.e. the one for inspiration and the one for expiration, resemble one another, be it that one curve increases while the other curve decreases (i.e. the trajectory is evolving (linearly) by means of the curves going up and down). The respective increasing and decreasing of the curves may evolve for example according to the same linearity or linear relation, as e.g. illustrated in Figure 2. It is noted however that, resembling or being comparable up to similar or equal in shape does not necessarily imply having the same slope, as e.g. shown in Figure 3. As the pressure linearly increases for a certain amount at inspiratory phase, the shape of the expiration curve approximates then a linear decrease. Without time off-set this linear decrease is for approximately the same amount and at the same pace, resulting in a perfect balance as represented by the triangle of Figure 2. In another embodiment of the invention, with time off-set, the linear decrease is adapted accordingly (after said time off-set). This latter embodiment is still (to be) interpreted as balanced (though not perfect), although representing less symmetry in the curve or profile as compared to the other/previous embodiment (with perfect balance).

The invention aims at balanced respiratory actuation, which can be described by one or more requirements:
1. Avoiding sudden changes in the air flow of the expiratory phase, which mathematically speaking results in the derivative over time of the flow rate being aimed to be small (no significant change) (possibly not during the transition from inspiration to expiration, and neither during expiration itself). By acting on (the beginning of) the expiration phase, e.g. be it with an time-off set, or during other/further part of the expiratory phase, or during both, for example by means of control actions being taken for inducing approximately a constant expiratory flow being equal to a constant inspiratory flow (see e.g. Figure 9), or equivalent thereto by letting the (airway) pressure (during the expiratory phase) being decreased linearly during this (expiratory) phase (and in line with the inspiratory phase).
2. Limiting or having a restriction on the expiratory flow and not on the pressure as typically performed in the art).
3. Approaching a flow during the expiratory phase which is comparable (not only in amount (integral action) but beyond that) (same shape for the profile) with the inspiratory flow.

As in the art (without use of the invention), the expiratory flow is rather large (and exponential) when compared to the inspiratory flow, the third requirement in accordance with the invention, which is an implementation choice, may result in the second requirement (for the invention). As in general, the inspiration flow is rather smooth, the first requirement may also be fulfilled.

According to an embodiment, the lowest possible inspiratory flow is taken at inspiration, in order to define the required volume, and the same flow is taken at expiration.

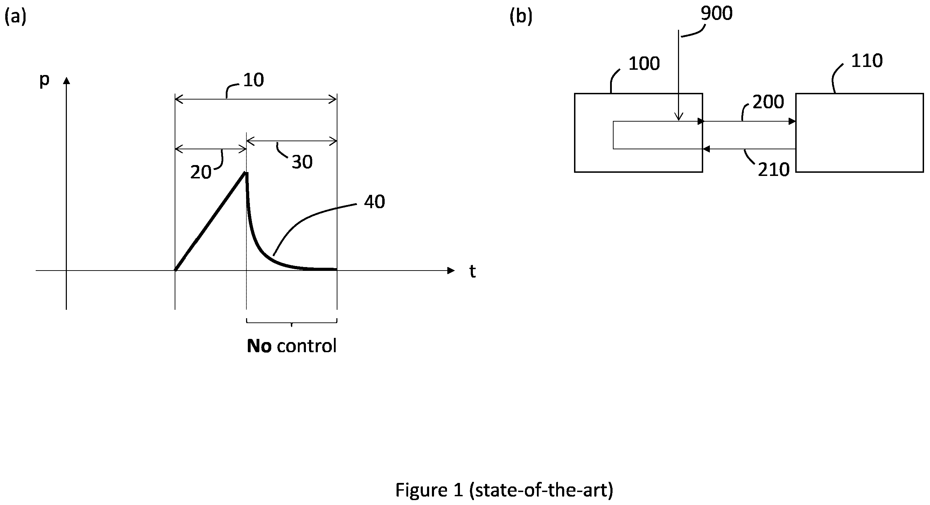

Figure 1 illustrates a graphical representation of the respiratory actuation of a patient as according to the art, wherein no active control is represented during the expiratory phase. It is noted that this graph is representative for Volume Controlled Ventilation (VCV), and does not apply for e.g. Pressure Controlled Ventilation (PCV), however PCV is not excluded from the invention. As shown in state-of-the-art curve depicted in Figure 1 (a), the respiratory actuation or ventilation of a patient 110, possibly an unconscious patient undergoing anaesthesia, wherein the patient being connected to a respiratory apparatus 100 for breathing e.g. during anaesthesia, has in one cycle 10 of such actuation, an inspiration part or inhaling sub-cycle or inspiratory phase 20 and an expiration part or exhaling sub-cycle or expiratory phase 30. The horizontal axis shows the time evolution while the vertical axis and the data line 40 are representing a parameter related to the respiration, e.g. the pressure in the lung (only schematically). It is worth noting at this point that, during the inhaling sub-cycle 20, the respiratory apparatus 100 - often also referred to as breathing apparatus or mechanical ventilator - forces by ventilation an airflow 200 into the patient's lung 110 while during the exhaling sub-cycle 30 the patient 110 induces an airflow 210 back to the respiratory apparatus 100 as schematically shown in Figure 1 (b). If the patient is sedated, this airflow inducing happens fully in a passive way, i.e. the patient's chest falls passively. Note that part of both airflows 200, 210 may use the same tubes (through which air is being conducted). Moreover, as due to the addition and hence presence of anaesthesia products 900 in the airflow 200, a closed air loop is required, in essence within the respiratory apparatus 100 recycling of (part of) the airflow 210 returning from the patient is provided for. In the state-of-the-art approach as illustrated here in Figure 1, the expiration or expiratory flow during the expiratory phase 30 is **not (actively) controlled** by the respiratory apparatus 100.

It is the purpose of the invention to provide an improved, and possibly or even preferably balanced, respiratory actuation or ventilation of a patient 110, by controlling the expiration during the expiratory phase 30 as illustrated in Figure 2. Figure 2 (a) shows the improved respiratory actuation of a patient 110, possibly being unconscious while undergoing anaesthesia, and being connected to a respiratory apparatus 100 for breathing e.g. during anaesthesia. Again, the respiratory actuation (although improved and hence slightly different as curve) is represented for one cycle 10 comprising an inspiratory phase 20 and an expiratory phase 30. It is noted that this controlling of the expiration may be interpreted as during essentially the whole of the expiratory phase 30, meaning for as good as whole but not necessarily during the entire expiratory phase 30. It may be for example that this controlling of the expiration is applied for a predetermined percentage of the expiratory phase instead, wherein typically this predetermined percentage is between 75% and 95%, more preferably between 80% and 90%. It is also possible for example that this controlling of the expiration is applied for about 90 or 95 percent of the expiratory phase 30 instead. The reason for this will be explained later. It is also noted that it can be interpreted that the inspiratory phase 20 is always controlled in some way, due to the active ventilation or supply of pressurized air to the patient 110. Such air supply can be set or tuned depending on the settings or parameters of the respiratory apparatus 100, which can be chosen and adjusted in relation to the patient (weight, age etc.) to be ventilated.

According to the invention, the controlling of the expiration can be applied by limiting the expiratory flow or return of pressurized air in the expiratory phase 30. This is indicated schematically in Figure 2 (b), wherein the respiratory apparatus 100 comprises a component 300, acting on the airflow 210 coming back from the patient 110. Due to the impacted airflow 210, also called the expiratory flow, the respiratory behaviour and in particular the expiratory phase 30 thereof is being affected as shown by data line 50 in Figure 2 (a). The component 300 is for example a membrane which can be pneumatically and/or electronically controlled. For the controlling of the expiration by limiting the expiratory flow or return of pressurized air in the expiratory phase 30, the respiratory arrangement for improved respiratory actuation of a patient, may be provided with a control system executing a control algorithm wherein first, the inspiratory airway pressure being monitored, and then based on the monitored inspiratory phase, the flow of pressurized air in the expiratory line being dynamically adjusted by controlling the component 300, for example a (pneumatically and/or electronically controlled) membrane, and hence the flow of pressurized air in the expiratory line can be dynamically adjusted by controlling e.g. the position of the membrane provided therein.

Figure 3 shows an embodiment of the invention, wherein the controlling of the expiration starts, not immediately at the start of the expiratory phase, but a while, here indicated as Δt, after the inspiratory phase ends. The respiratory behaviour and in particular the expiratory phase 30 thereof is being affected as shown by data line 60 in Figure 3 (a). Because of this slight delay Δt in control during the expiratory phase, it becomes clear that this embodiment can be seen as an example wherein the controlling of the expiration is applied for a vast majority percentage (and thus not 100%) of the expiratory phase 30. Hence, the earlier notification that the controlling of the expiration may be interpreted as during essentially the whole of the expiratory phase 30 (and not necessarily during the entire expiratory phase 30), is herewith further explained and illustrated.

Some state-of-the-art respiration approaches do foresee precautionary measures such that the lung or airway pressure, at the (very) end of the respiration cycle, doesn't drop below a certain threshold.

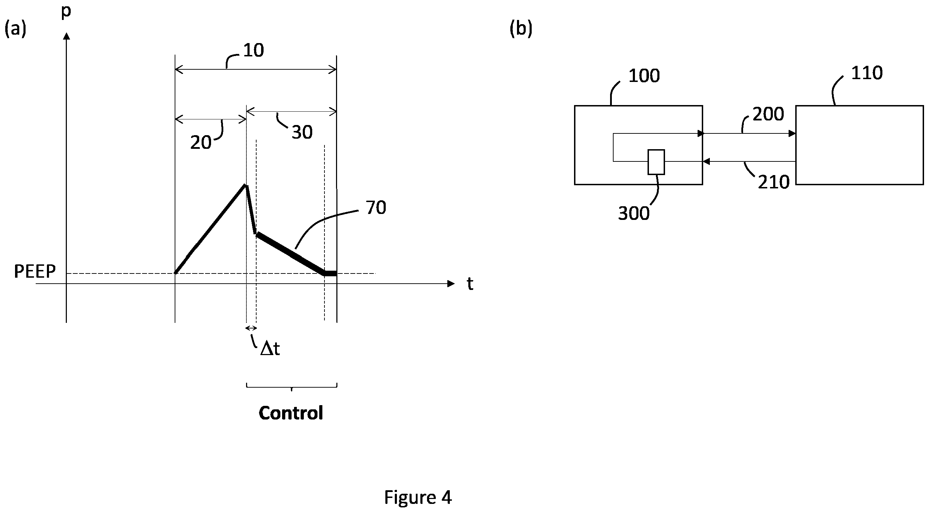

Obviously this occurs de facto during the expiratory phase of the respiration cycle to attain a certain minimum value of pressure and does not lead to a more improved (balanced) flow or respiratory actuation under (active) control of the respiratory apparatus 100 during (essentially the whole of) the expiratory phase 30 for controlling the expiration. Herewith is referred to commonly known PEEP (Positive End Expiratory Pressure) in the art. Figure 4 illustrates an embodiment of the invention, taking into account the provision of this generally known PEEP. The respiratory behaviour and in particular the expiratory phase 30 thereof is being affected now as shown by data line 70 in Figure 4 (a). The earlier notification that the controlling of the expiration may be interpreted as during essentially the whole of the expiratory phase 30 (and not necessarily during the entire expiratory phase 30), is herewith even further motivated. It is however noted that during expiration, an intrinsic PEEP can be evaluated and avoided where possible by dynamically (breath-over-breath) adjusting the balancing parameters to release air within the breath. This means that at for example 80% or 90% of the time of the expiration, we no longer exert any regulation or so-called back pressure on the exhalation to ensure that the air is exhaled effectively and no (additional) pressure build-up in the lungs can occur.

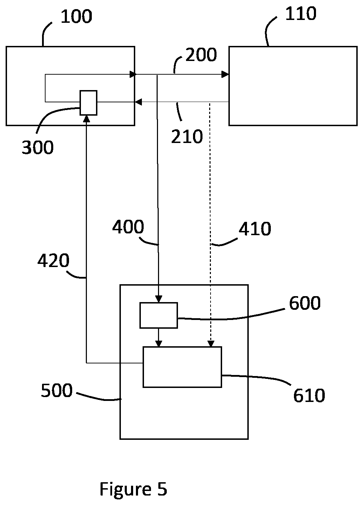

As shown in the embodiment of Figure 5, the improved (balanced) flow under (active) control of the respiratory apparatus 100 is based on measurements 400 taken during the inspiratory phase 20, for monitoring the inspiratory behaviour, and the controlling of the expiration during the expiratory phase 30 is based on this monitored inspiratory behaviour. Further shown in Figure 5 is a controller 500 comprising storage means 600. The controller 500 is receiving the measurements 400 from the inspiratory line and provides control signals 420 to the respiratory apparatus 100. While with improved flow is meant that the total respiratory behaviour should from treatment perspective be better than a traditional approach as according to the art, wherein in essence the expiratory phase is left uncontrolled (except for some threshold control), we now proceed with the fact that the improvement can be realized by adopting the notion of balanced flow, in that the aim is to sort of ensure some symmetry with the inspiratory phase. It is hence important to note that the measurements 400 are not instantaneously used (meaning at the time of measurement) but can be first stored in the storage means 600 as part of the controller 500, to then further be used for determining or computing the control signals 420, for example by means of computation means 610 as also illustrated here. While in an embodiment of the invention the feedforward control based on measurements 400 may be used, in a more preferred embodiment, also measurements 410, indicated by the dashed line, are taken during the expiration flow, i.e. from the expiratory line, are used (in essence now instantaneously) in the determining or computing of control signals 420.

The embodiment of Figure 4 (with end pressure control (PEEP)) can easily be integrated in the embodiment of Figure 5 including preferred aspect thereof with measurements 410 taken from the expiratory line. Also the embodiment of Figure 3 (with delayed start of the active control) can benefit from the use of measurements 410 in that the activation can be derived from those. It is worth noting that besides the end pressure control, during the respiration cycle, the (lung or airway) pressure is steered or controlled by the controller towards a predetermined trajectory, itself based on the measurement 400. In essence a mixed feedforward feedback control principle is hence adopted. Note that with non-instantaneously is meant that the capturing is done at a different cycle than the one wherein the measurement is used. With instantaneously is meant in essence direct use, but an embodiment wherein a plurality of measurements are taken, for instance for data manipulation handling like filtering, averaging (and hence requiring also temporal storage) can also be included herein.

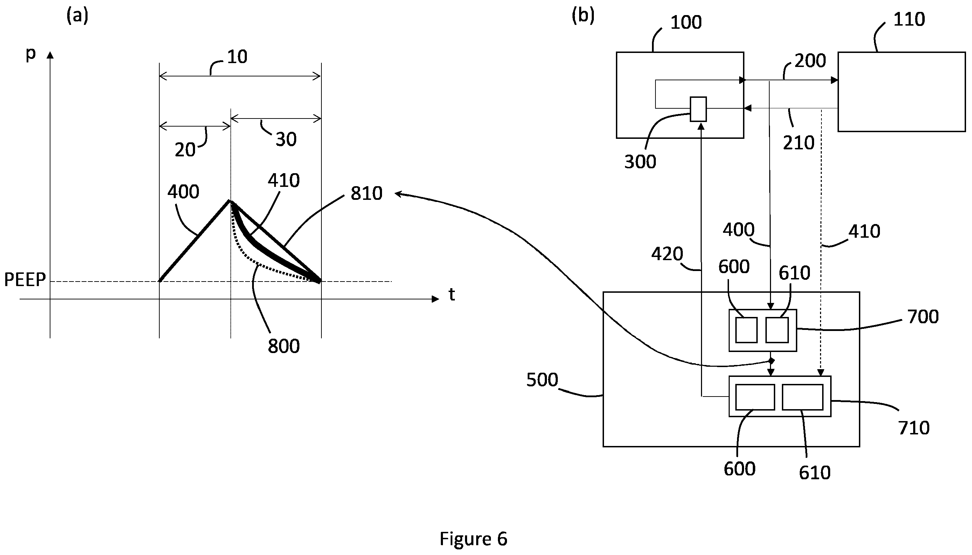

Figure 6 gives an alternative view on the invention. While Figure 5 provides a schematic view on the hardware requirements of the controller 500, i.e. storage means 600 or memory and computing facilities 610, Figure 6 (b) provides an illustration of the feedforward controller 700 and the feedback controller 710 combination, wherein in essence each may have some storage means 600 and computing facilities 610. Figure 6 (a) shows the measurements in relation to the time dynamics, in that measurements 400 are taken during sub-cycle 20 while the measurements 410 are captured during sub-cycle 30. Moreover, the effect is shown in that dotted curve 800 represents the case wherein no control is done, whereas curve 810 shows what one would like to achieve (based on the measurements 400) and hence this is also the output of the feedforward controller 700, while the measurements 410 is what is actually measured in Figure 6 (a) and hence what is achieved as curve in Figure 6 (b) by the control.

The embodiment of Figure 7 illustrates that the step of controlling the expiration, in the method for providing an improved respiratory actuation of a patient, may only start after a predetermined amount of inspiration/expiration cycles 80 for a time tᵢ, wherein such time tᵢ being used for initialization and/or for letting the respiratory arrangement (including respiratory apparatus 100 and patient 110) stabilize. During this time tᵢ, the initialization parameters may be measured or determined which can be used for the control algorithm. Moreover, if the inspiration/expiration cycles 80 run smoothly, one may decide not to activate the control. On the other hand, in the alternative that the cycles show control may be required, one may by use of monitoring these cycles, possibly automatically, activate the control instead.

Note that in the above the control objective is formulated and/or described with respect to pressure. Obviously, also other measurements can be used such as flow rate. Even more preferred, a combination of those control objectives are considered.

The present invention is defined in independent claim 1. Preferred embodiments are matter of the appended dependent claims.

## Claims

1. A system for providing a balanced respiratory actuation of a patient (110), being connected to a respiratory apparatus (100) for breathing, the respiratory actuation comprising an inspiratory and an expiratory phase (20, 30) during which respectively inspiration and expiration of the patient (110) takes place, wherein the system comprises a controller for executing a control algorithm (i) monitoring the inspiratory airway pressure and herewith defining a trajectory for the inspiratory phase, and (ii) based thereon, dynamically adjusting the flow of pressurized air in the expiratory line, herewith controlling the lung or airway pressure towards a predetermined trajectory,
wherein said predetermined trajectory being based on the monitored inspiratory airway pressure and resembling in shape the trajectory of the inspiratory phase.

2. The system of claim 1, wherein dynamically adjusting the flow of pressurized air in the expiratory line is performed by pneumatic means and/or electronic means.

3. The system of claim 1 or 2, wherein dynamically adjusting the flow of pressurized air in the expiratory line is performed by means of controlling the position of a membrane (300) provided therein, or based on the actuation of a proportional valve.

4. The system of claim 1 to 3, wherein the controller for executing a control algorithm is also taking measurements during the expiration phase, and said dynamically adjusting the flow of pressurized air in the expiratory line is based on said expiration measurements.

5. The system of claim 1 to 4, wherein the controller for executing a control algorithm starts after a set and/or computed time period and/or a certain condition is satisfied after the inspiration ends.

6. The system of claim 1 to 5, wherein the controller for executing a control algorithm stops after a set and/or computed time period and/or a certain condition is satisfied after the inspiration ends.

7. The system of claim 1 to 6, wherein the lung or airway pressure being controlled towards a predetermined trajectory, is **characterized by** a linear decrease.

## Patentansprüche

1. System zum Bereitstellen einer ausgeglichenen Atmungsbetätigung eines Patienten (110), das zum Atmen mit einem Beatmungsgerät (100) verbunden ist, die Atmungsbetätigung umfassend eine Inspirations- und eine Exspirationsphase (20, 30), während derer jeweils die Inspiration und Exspiration des Patienten (110) stattfindet, wobei das System ein Steuerung zum Ausführen eines Steueralgorithmus umfasst, der (i) den Inspirationsdruck in den Atemwegen überwacht und hiermit eine Trajektorie für die Inspirationsphase definiert, und (ii) darauf basierend den Druckluftstrom in der Exspirationsleitung dynamisch anpasst, wobei hiermit der Lungen- oder Atemwegsdruck in Richtung einer zuvor bestimmten Trajektorie gesteuert wird, wobei die zuvor bestimmte Trajektorie sich auf dem überwachten Inspirationsdruck in den Atemwegen basiert und in ihrer Form der Trajektorie der Inspirationsphase ähnelt.

2. System nach Anspruch 1, wobei das dynamische Anpassen des Druckluftstroms in der Exspirationsleitung durch pneumatische Mittel und/oder elektronische Mittel durchgeführt wird.

3. System nach Anspruch 1 oder 2, wobei das dynamische Anpassen des Druckluftstroms in der Inspirationsleitung mittels dem Steuern der Position einer darin bereitgestellten Membran (300) oder basierend auf der Betätigung eines Proportionalventils durchgeführt wird.

4. System nach Anspruch 1 bis 3, wobei die Steuerung zum Ausführen eines Steueralgorithmus ebenso während der Exspirationsphase Messungen vornimmt und das dynamische Anpassen des Druckluftstroms in der Exspirationsleitung sich auf den Exspirationsmessungen basiert.

5. System nach Anspruch 1 bis 4, wobei die Steuerung zum Ausführen eines Steueralgorithmus nach einer festgelegten und/oder berechneten Zeitspanne startet und/oder eine bestimmte Bedingung nach dem Ende der Inspiration erfüllt ist.

6. System nach Anspruch 1 bis 5, wobei die Steuerung zum Ausführen eines Steueralgorithmus nach einer festgelegten und/oder berechneten Zeitspanne stoppt und/oder eine bestimmte Bedingung nach dem Ende der Inspiration erfüllt ist.

7. System nach Anspruch 1 bis 6, wobei, dass der Lungen- oder Atemwegsdruck in Richtung einer zuvor bestimmten Trajektorie gesteuert wird, **gekennzeichnet ist durch** eine lineare Abnahme.

## Revendications

1. Système permettant de fournir un actionnement respiratoire équilibré d'un patient (110), étant relié à un appareil respiratoire (100) pour la respiration, l'actionnement respiratoire comprenant une phase inspiratoire et une phase expiratoire (20, 30) pendant lesquelles l'inspiration et l'expiration du patient (110) ont lieu respectivement, dans lequel le système comprend un dispositif de commande pour l'exécution d'un algorithme de commande (i) surveillant la pression inspiratoire des voies respiratoires et définissant ainsi une trajectoire pour la phase inspiratoire, et (ii) sur la base de celle-ci, l'ajustement dynamique du flux d'air sous pression dans la ligne d'expiration, en commandant la pression des poumons ou des voies respiratoires vers une trajectoire prédéterminée, dans lequel ladite trajectoire prédéterminée est basée sur la pression des voies respiratoires inspiratoires surveillée et ressemblant par sa forme à la trajectoire de la phase inspiratoire.

2. Système selon la revendication 1, dans lequel l'ajustement dynamique du débit d'air sous pression dans la ligne expiratoire est effectué par un moyen pneumatique et/ou électronique.

3. Système selon la revendication 1 ou 2, dans lequel l'ajustement dynamique du débit d'air sous pression dans la ligne expiratoire est effectué par la commande de la position d'une membrane (300) fournie à cet effet, ou sur la base de l'actionnement d'une valve proportionnelle.

4. Système selon la revendication 1 à 3, dans lequel le dispositif de commande pour l'exécution d'un algorithme de commande prend également des mesures pendant la phase d'expiration, et ledit ajustement dynamique du flux d'air sous pression dans la ligne d'expiration est basé sur ces mesures d'expiration.

5. Système selon la revendication 1 à 4, dans lequel le dispositif de commande pour l'exécution d'un algorithme de commande démarre après une période de temps définie et/ou calculée et/ou une certaine condition est satisfaite après la fin de l'inspiration.

6. Système selon la revendication 1 à 5, dans lequel le dispositif de commande pour l'exécution d'un algorithme de commande s'arrête après une période de temps définie et/ou calculée et/ou une certaine condition est satisfaite après la fin de l'inspiration.

7. Système selon la revendication 1 à 6, dans lequel la pression des poumons ou des voies respiratoires étant contrôlée vers une trajectoire prédéterminée, est **caractérisée par** une diminution linéaire.
